# EUROPEAN PATENT APPLICATION

(11) **EP 2 868 322 A1**
(43) Date of publication of application: **06.05.2015**
(21) Application number: 14168550.3
(22) Date of filing: 15.05.2014
(51) Int. Cl.: A61K 31/56, A61P 5/46

(54) **A composition or group of compositions for use in a method for inhibiting autocrine HCG production in adult human cells**

(30) Priority: 03.11.2013 EP 13191320
(71) Applicant: Flamina Holding AG, 6304 Zug (CH)
(72) Inventor: May, Michael, CH-6304 Zug (CH)
(74) Representative: Rutz & Partner

(57) **Abstract**

The composition for inhibitting autocrine HCG (Human Chorionic Gonadotropin) production in adult human cells, by administration of a composition containing at least one active component, said active agent being a competitively binding progesterone antagonist competitively binding to steroid receptors of human cells.

## Description

The present invention relates to a composition or a group of compositions for use in a method for inhibiting autocrine production of HCG (Human Chorionic Gonadotropin) in cells of adult human organisms.

### BACKGROUND OF THE INVENTION

According to [1], Marie Tsampalas et al., "Human chorionic gonadotropin: A hormone with immunological and angiogenic properties", 2010 Elsevier Ireland Ltd., the mechanisms underlying human implantation and particularly immune tolerance of pregnancy still remain to be defined in detail. HCG is the prime mediator by which the embryo announces its presence to the maternal organism since it is produced even before implantation. Among the wide range of mediators present at the implantation site, a role is becoming evident for HCG as specific blastocyst signal involved in orchestrating the implantation cascade. HCG is implicated in several actions that promote immune tolerance and angiogenesis and thus has physiological important implications for successful pregnancy. HCG is one of the earliest molecules produced by the embryo. Indeed, its mRNA is transcribed as early as the 8-cell stage and the blastocyst produces the protein before its implantation. HCG is increasingly produced after implantation by the syncitiotrophoblast. Significant levels of HCG can already be measured in the maternal blood 10 days after ovulation. The peak of HCG production by the placenta is reached between the 10th and the 11th week of gestation, then the production decreases at the 12th week to remain at low levels for the remainder of pregnancy. HCG affects the corpus luteum to prevent luteolysis and favour stimulation of progesterone production.

HCG production is stimulated by the producing cell itself, it is known as autocrine hormone production that takes place during the above described embryonic phase. This process of autocrine hormone secretion by embryonic cells is stopped before parturition. From then on, all hormone production is controlled by the brain. After the specified initial time period HCG is no longer produced in genetically healthy adult human cells.

By determination, the descendants of the totipotent zygote successively lose their totipotenz and follow their predetermined determination. Remarkably, each healthy cell has a cell-memory, retaining its own cell proliferation, a necessity for organized tissues, organs and stably differentiated cell types.

The presence of HCG in the human body can be measured. Pregnancy testing for example is based on the measurement of the concentration of HCG contained in the maternal urine.

As generally known, HCG interacts with the Lutheneizing hormone (LH) receptor of the mother and promotes the maintenance of the corpus luteum during the beginning of pregnancy. This allows the corpus luteum to secrete the hormone progesterone, which enriches the uterus with blood vessels and capillaries so that it can sustain the growing fetus. Consequently, the application of competitively binding progesterone antagonists induces an abortion, since they occupy the receptor of the progesterone controlling gene, that functions to maintain pregnancy.

As described in [2], B. Maria et al., "Termination of early pregnancy by a single dose of mifepristone (RU486), a progesterone antagonist", European Journal of Obstetrics & Gynecology and Reproductive Biology, vol. 28, no. 3, pages 249-255, (July 1, 1988), the compound RU486, also known as Mifepristone, is an important progestin antagonist that effectively and safely terminates early pregnancy, when used in conjunction with a prostaglandin.

Derivatives or descendants of RU486 are described in [3], US6608074B2 and [4], US6316432B1.

As described in [5], "New Zealand Data Sheet of Mifegyne", 3 July 2013, XP855181287, Mifepristone is commercially available in tablets of 200 mg under the trade name Mifegyne®. Mifepristone is a synthetic steroid with an antiprogestational action as a result of competition with progesterone at the progesterone receptors. As further described, doses higher or equal to 1 mg/kg, mifepristone antagonises the endometrial and myometrial effects of progesterone.

In [6], EP2210585A1, compositions with an antiprogestational action are described, which have the ability to prevent the intake of the composition by the patient in an abortive amount and avoid their misuse.

As detailed above, HCG fulfils important functions in the initial phase of human life but is no longer needed at a later stage. Cells need to be able to switch genes ON and OFF individually, e.g. in order to coordinate cohabitation with neighbour cells or to ensure that hormones required for specific functions are present or not. After HCG has fulfilled its purpose, the gene responsible for HCG production is switched OFF and the related nuclear receptors are no longer activated and transcription is ceased.

Mechanisms of action of nuclear receptors are described in [7], Aranda et al., "Nuclear Hormone Receptors and Gene Expression", PHYSIOLOGICAL REVIEWS, Vol. 81, No. 3, July 2001. For example, a lipophilic hormone may diffuse through the membrane of a cell to the cytosol. Once in the cytosol, the hormone binds to its cytosolic receptor, causing the release of an inhibitory protein from the receptor. The activated receptor then diffuses into the nucleus. In the nucleus, the receptor-hormone complex binds to the enhancer regions of hormone-regulated genes and transcription of the genes is stimulated, i.e. the gene is switched on.

While the function of HCG during the initial part of the embryonic phase is rather well known, potential effects of HCG in the post embryonic phase are not explored.

In [8], M.J. Duffy and P. McGing, "Guidelines for the Use of Tumour Markers", Scientific Committee of the Association of Clinical Biochemists in Ireland (ACBI), 3rd Edition, April 2005, HCG is known as a Tumour Marker, which occurs in elevated levels in the event that a patient suffers from malignancies such as different kind of tumours. HCG is therefore used as an indicator for a malignancy.

Investigations indicate that in extremely seldom cases, a gene required for hormone production, which should be switched OFF, is accidentally switched ON and the related hormone is produced. However, this accidental status change of the cell is not without reason or cause.

It is known that a cell can adapt to external conditions. Such adaptations are typically reversible changes in the number, size, phenotype, metabolic activity, or functions of cells in response to changes in their environment. Physiologic adaptations usually represent responses of cells to normal stimulation by hormones or endogenous chemical mediators. Pathologic adaptations are responses for example to stress that allow cells to modulate their structure and function and thus escape injury. Such adaptations can take several distinct forms. In [9], Thomas Yuill, "Cellular adaptation to toxicity", National Library of Medicine, October 2006, it is described that adaptive changes often result in cells or organs that cannot function normally. This imperfect adaptation is a pathological change.

Hence, depending on environmental conditions and physiological and psychological influences or a mixture thereof to a human body, the cell system may be stressed and weakened and cells that suffer from pathological changes are prone to malfunction. Such an occurrence may be compared to an apparently inexplicable street accident, in which the driver has possibly actuated the accelerator pedal instead of the brake pedal. In such cases the driver has also been under the influence of psychological or environmental disturbances, which are more critical if the driver suffers from a weakness or illness at the same time. Similarly, a cell in a pathological state appears to act paradoxically at first sight. However, unfortunately, unlike the car driver, the cell will not encounter an adequate response to the malfunction but will remain under the impression that the selected state, with the undesirable gene transcription switched on, is correct.

Hence, since some people will always be exposed to conditions, in which extensive cell adaptations over a longer period of time occur, e.g. by consumption of unhealthy nutrition, lack of physical exercises or disturbing influences, such as stress or unclean air, deseases will always occur that have the origin in the pathological state of cells located in a part of the human body.

### SUMMARY OF THE INVENTION

Consequently, in the event that a gene that is required for HCG-production and that is present in a disturbed or stressed adult cell is accidentally switched ON due to a cell weakness or irritating signalling, then autocrine production of HCG will start. It has been assumed before that HCG is a symptom or indicator of a malignancy.

In [10], Laurence A Cole, "hCG, the wonder of today's science" Reproductive Biology and Endocrinology 2012, http://www.rbej.com/content/10/1/24, it is stated that cancers start out as transformed cells driven by an HCG-independent process. Only after the cancer progresses and becomes advanced it would become able to express the HCG β-subunit gene and make HCG free ß-subunit. The HCG free ß-subunit driven TGFß antagonism mechanism then would take over control of the cancer, as indicated by vaccine studies, and would reach complete control of the advanced disease.

In contradiction to this statement, applicant has discovered by further studies that the development of malignant cancer in otherwise healthy cells starts by autocrine HCG-production. In other words, start of autocrine HCG-production leads to oncogenesis.

Tests in cell cultures show that nascent HCG from early embryonic of vertebrates, in particular mamifers, promotes cancerous cell growth. If the fresh cells have been given the equivalent of 0.5 mg per kg of a progesterone antagonist, the above-mentioned doping with HCG did not result in cancerous cell growth.

By the accidentally started autocrine HCG-production, the process of programmed cell death (PCD), apoptosis, is impaired. Since apoptosis is a critical maintenance function of the cell system that causes the death and safe removal of more than 50 billion deficient cells each day, even partial and local disturbances of the apoptosis-function are undesirable. While HCG is contributing to the condition required for the generation of life, it has been found harmful for the important process of the destruction of undesirable cell life, for which proliferation should be inhibited.

Besides a deregulation of the apoptosis function, autocrine HCG production will lead to impairment of the immune defence system, to hormonal disorders and reduced efficiency, effect or accuracy of the DNA repair mechanism of the cell. In adult cells, HCG molecules resulting from autocrine production facilitate activities of endogenous of exogenous agents that cause DNA damages, which over years, due to disturbances of the apoptosis function, can increase and then can be the origin of a variety of diseases in the human body after many years.

Based on the foregoing, the primary object of the present invention is providing a composition or a group of compositions for use in a method for inhibiting the occurrence of intra-cellular disorders in post natal or adult human organs, particularly cellular disorders in stressed cells.

Cells, particularly stressed cells, which are exposed to damaging influences shall be immunised against pathological cell behaviour and irritating signalling.

More particular, the invention is based on the object of preventing cell disorders that are caused and supported by the occurrence of autocrine HCG production in post-natal or adult cells.

Further, the composition shall be used in a method used for treatment of pathological cells and for inhibiting cell disorders causing diseases such as a deregulation of the immune defence system, hormonal disorders, reduced efficiency and effect or accuracy of DNA repair and impairment and foremost disturbances of the apoptosis function.

It is another object of the invention to provide such a composition or a group of compositions at comparatively low cost, with high efficiency and free of undesirable side-effects.

The above and other objects of the present invention are achieved with a composition according to claim 1 and a group of compositions according to claim 10.

### DESCRIPTION OF THE INVENTION

The real possibility that a gene of an adult cell accidentally, but not without cause, reverts partially into an early embryonic state has not yet been considered seriously. This explains why no attempts to counteract such an effect have been explored.

Under real life conditions, an eventual and very rare accidental start of autocrine HCG-production in an adult human cell needs several years to eventually develop a detectable disease, for which HCG would be used as a suitable indicator.

Possibly an autocrine HCG producing cell can under some conditions, pervert neighbouring cells, thereby spreading and accelerating the propagation of the abnormal functioning of its gene.

Hence, the present invention is targeting the prevention of autocrine HCG production, for which a HCG antagonist or a substance with similar effect is used.

In [10], five unique variants of HCG molecules are described, each having identical amino acid sequence, produced by different cells and having independent functions. These are HCG, sulfated HCG, hyperglycosylated HCG, HCG free ß-subunit and hyperglycosylated HCG free ß-subunit. It is further stated that based on the vaccine studies it has been concluded, that development of a human high affinity HCG ß-subunit antibody may be a future answer to human cancer treatment. At the extraordinarily mature state of the actual Endocrinology research and activity it has been a particular surprise to discover that the long known competitively binding progesterone antagonist is also able to antagonise the five variants of HCG molecules. Up to now, progesterone antagonists have been used to terminate an early pregnancy.

It could be shown, that surprisingly even a very low amount of a competitively binding progesterone antagonist as active agent is able to inhibit unwanted autocrine HCG production in adult cells, thus eliminating the risk of potential damage caused by this unwanted process.

Extrapolations of in vitro experiments indicate that already 0.1 mg to 2 mg per kg body weight and per year, preferably 0.5 mg to 1 mg per kg body weight and per year of the active agent are sufficient.

It has thus been a surprise to realize, that a progesterone antagonist is able to counteract and inhibit the autocrine HCG production of an adult cell.

The invention comprises therefore two revolutionary steps. In the first step an important cause of oncogenesis as well as a solution for its suppression has been found, namely the application of extremely small amounts of HCG antagonists. In a second step it has been found that a competitively binding progesterone antagonist is suitable for antagonising the receptors, i.e. occupying the receptors with an antagonist, within the cells that produce the HCG-forms described in [10].

Preferably the composition is applied with low volumes in intervals. Since the composition is applied for preventive purposes the required concentration is very low. A time spacing of approximately 3 to 24 months, preferably approximately 6 months, between applications showed good results in preliminary testing.

It is however recommended to administer a higher dose of the composition after a number of intervals. E.g., lower doses are applied at three months intervals while every eighteenth month a dosage is applied that is higher compared to the normal monthly dose by a factor in the range between 1.25 and 5. This factor is typically dependent of the amount of the monthly dose and the region of application. E.g., if the dosage is in the range of 0.1 mg to 0.5 mg per kg body weight and per year, then the maximum multiplication factor can be taken.

The active agent is preferably combined with at least one pharmaceutically acceptable carrier medium or excipient in order to improve the transport of the molecules of the active agent into the cytosol of the cells. The weight ratio of the active agent to the carrier substance is preferably in the range between 1:10 and 1:250, most preferably at 1:33. The ratio is preferably selected according to the frequency of application. In the event that an application is made once a year, then a higher density in the range of 1:10 to 1:33 is seleceted.

It has been found that compositions are advantageously applicable that have been developed for contraceptive purposes such as the compounds disclosed in [3], US6608074B2 and [4], US6316432B1. Suitable progesterone antagonists are for example:
a) 11β-[4-N,N-(dimethylamino)phenyl]-17β-hydroxy-17α-propinyl-estra-4,9-dien-3-one, (RU486 or Mifepristone);
b) 11β[(4-N,N-Dimethylamino)-phenyl]-17β-hydroxy-17α-propinyl-4,9(10)-estradien-3-one;
c) 11β-[(4-N,N-dimethylamino)-phenyl]-17β-hydroxy-18-methyl-17α-propinyl-4,9(10)-estradien-3-one;
d) 11β-(4-N,N-dimethylamino)-phenyl]-17aβ-hydroxy-17aα-propinyl-D-homo-4,9(10),16-estratrien-3-one;
e) 11β-p-methoxyphenyl-17β-hydroxy-17α-ethinyl-4,9(10)-estradien-3-one;
f) 11β-(4-acetylphenyl)-17β-hydroxy-17α-(prop-1-inyl)-4,9(10)- estradien-3-one;
g) 11β-(4-Dimethylaminophenyl)-17α-hydroxy-17β-(3-hydroxypropy 1)-13α-methyl-4,9-gonadien-3-one;
h) 11β-(4-acetylphenyl)-17β-hydroxy-17α-(3-hydroxyprop-1-enyl)-4,9(10)-estradien-3-one;
i) 11β,19-[4-(cyanophenyl)-o-phenylenel-17β-hydroxy-17α-(3-hyd roxyprop-1(Z)-enyl)-4-androsten-3-one;
j) 11β,19-[4-(3-pyridinyl)-o-phenylene]-17β-hydroxy-17α-(3-hyd roxyprop-1(Z)-enyl)-4-androsten-3-one; or
k) 11β-[(4-N,N-dimethylamino)-phenyl]-17α-hydroxy-17β-(3-hydroxypropyl)-13α-methyl-4,9(10)-gondien-3-one
l) Aglepristone (RU534).

The first compound is RU486 or Mifepristone, which can be used as active agent as well as substituents thereof.

The last compound is RU534 or Aglepristone is known from [11], F. Fieni et.al., "Mid-pregnancy termination in bitches with an antiprogestin: aglepristone (RU534)", published in "Advances in dog, cat and exotic carnivore reproduction", Oslo 2000.

The annual dosage of the composition, applied annually, semi-annually or monthly, is selected in the range between 0.1 mg to 2.0 mg per kg of person/body-weight, preferably 0.5 mg to 1.0 mg per kg of person/body-weight. The higher dosage being recommended for males and post-menopausal women

For females in the childbearing years, the annual dosage of the composition, preferably applied weekly or monthly, is limited to 0.1 mg per kg of person/body-weight.

Still further, the dosage is preferably adapted in accordance with environmental, habitual and genealogical considerations. In the event that the patient is exposed to stress and/or a genealogical handicap, then the dosage is preferably increased by a factor in the range of 2 to 5. Thus, the dosage and consequently the costs and efforts for the application can be optimised and kept extremely low, while the benefit is substantial.

For males and females above the age of 45 the above specified values of the dosage of application can be elevated by the factor 2-5.

According to the invention the absorption of the active agent is improved if a pharmaceutically acceptable first carrier medium is combined with the active agent that facilitates the transport of the molecules of the active agent into the cytosol of the cells.

Good results are achieved if the first carrier medium is a carrier protein, a cell penetrating peptide, a polar aprotic solvent such as Dimethyl sulfoxide (DMSO), a lipid, an alcohol, a permease, a liposome, a sulfur containing compound or a mixture thereof.

A safe and controllable application of the active agent is obtained, if a second pharmaceutically acceptable carrier medium is added to the composition, as for example a liquid carrier, if the active agent shall be injected, a gelatin for sublingual application, a suppository for anal, a cream, spray or oil for mucosal or transdermal application and a syrup or substance for producing pills or capsule for oral application.

For treatments of an aggravated deficiency of the epithelium, second carrier products, as creams, spray, suppository, transdermal patches can be useful to facilitate applications comprising 1 w%/W to 10 w%/W, preferably 2 w%/W to 5 w%/W of the active agent and the at least one carrier.

Hence, inhibiting autocrine HCG-production can be performed with low dosages that exhibit no side effects. Further, costs are extremely low even for general preventive application in the public. Potential risks are avoided or significantly reduced, which could lead to high healing costs and suffering of the patients.

Preferably a group of at least a first and a second composition is provided that comprise different carrier media and that are applicable in different parts of the body for forwarding the active agent via different pathways to a predetermined application zone.

Preferably the first composition that is provided with a first concentration of the active agent and the second composition that is provided with a second concentration of the active agent are adapted to one another in such a way that the desired amount of active agent is delivered to the field of application and the concentration of the active agent along a specific pathway does not exceed specified values. The first composition is provided for example for transdermal application and the second composition is provided for inhalatory application. Alternatively the first composition is provided for transdermal application and the second composition is provided for oral application. For females concentrations internally applied in the region of the lower abdomen are preferably lower by a factor in the range from 2 to 5 compared to a composition applied e.g. transdermally in the in the breast region.

Consequently, the invention allows to 1 advantageously apply the inventive composition adapted to the sex, the weight, the age, the genealogy, and the physical and psychological condition of the patient without inducing side effects.

### References

[1] Marie Tsampalas et al., "Human chorionic gonadotropin: A hormone with immunological and angiogenic properties", 2010 Elsevier Ireland Ltd.
[2] B. Maria et al., "Termination of early pregnancy by a single dose of mifepristone (RU486), a progesterone antagonist", European Journal of Obstetrics & Gynecology and Reproductive Biology, (1988-07-01), vol. 28, no. 3, pages 249-255
[3] US6608074B2
[4] US6316432B1
[5] "New Zealand Data Sheet of Mifegyne", 3 July 2013, XP855181287
[6] EP2210585A1,
[7] Aranda et al., Nuclear Hormone Receptors and Gene Expression, PHYSIOLOGICAL REVIEWS, Vol. 81, No. 3, July 2001
[8] M.J. Duffy and P. McGing, Guidelines for the Use of Tumour Markers, Scientific Committee of the Association of Clinical Biochemists in Ireland (ACBI), 3rd Edition, April 2005
[9] Thomas Yuill, Cellular adaptation to toxicity, National Library of Medicine, October 2006
[10] Laurence A Cole, "hCG, the wonder of today's science" Reproductive Biology and Endocrinology 2012, http://www.rbej.com/content/10/1/24
[11] F. Fieni et.al., "Mid-pregnancy termination in bitches with an antiprogestin: aglepristone (RU534)", Laborarory of Biotechnology and Pathology of Reproduction, National Vererinary School, Nantes, France; published in "Advances in dog, cat and exotic carnivore reproduction", Oslo 2000

## Claims

1. Composition for use in a method for inhibiting autocrine HCG production in adult human cells in order to avoid the occurrence of deficiencies of cell functions relating to impaired apoptosis, impaired immune defence and impaired DNA repair, by the administration of a composition containing at least one active component, said active agent being a competitively binding progesterone antagonist binding to steroid receptors of human cells.

2. Composition according to claim 1, whereby the active agent is RU486 or RU534 or a substituent thereof.

3. Composition according to claim 1, whereby the active agent is **characterised by** the formula
- 11β[(4-N,N-Dimethylamino)-phenyl]-17β-hydroxy-17α-propinyl-4,9(10)-estradien-3-one;
- 11β-[(4-N,N-dimethylamino)-phenyl]-17β-hydroxy-18-methyl-17α-propinyl-4,9(10)-estradien-3-one;
- 11β-(4-N,N-dimethylamino)-phenyl]-17aβ-hydroxy-17aα-propinyl-D-homo-4,9(10),16-estratrien-3-one;
- 11β-p-methoxyphenyl-17β-hydroxy-17α-ethinyl-4,9(10)-estradien-3-one;
- 11β-(4-acetylphenyl)-17β-hydroxy-17α-(prop-1-inyl)-4,9(10)- estradien-3-one;
- 11β-(4-Dimethylaminophenyl)-17α-hydroxy-17β-(3-hydroxypropy 1)-13α-methyl-4,9-gonadien-3-one;
- 11β-(4-acetylphenyl)-17β-hydroxy-17α-(3-hydroxyprop-1-enyl)-4,9(10)-estradien-3-one;
- 11β,19-[4-(cyanophenyl)-o-phenylene]-17β-hydroxy-17α-(3-hyd roxyprop-1(Z)-enyl)-4-androsten-3-one;
- 11β,19-[4-(3-pyridinyl)-o-phenylene]-17β-hydroxy-17α-(3-hyd roxyprop-1(Z)-enyl)-4-androsten-3-one; or
- 11β-[(4-N,N-dimethylamino)-phenyl]-17α-hydroxy-17β-(3-hydroxypropyl)-13α-methyl-4,9(10)-gondien-3-one;
or derivatives thereof.

4. Composition according to claim 1, 2 or 3, whereby the composition is provided in units designed for the monthly, semi-annual or annual application of the active agent in annual dosages in the range between 0.1 mg to 2.0 mg per kg of person/body-weight, preferably 0.5 mg to 1.0 mg per kg of person/body-weight.

5. Composition according to claim 1, 2 or 3, whereby the composition is provided in consumable units containing the active agent with a dosage or concentration that is below the implantation inhibiting dose.

6. Composition according to one of the claims 1 - 5, whereby a pharmaceutically acceptable first or a second carrier medium that enables the transport of the active molecules of the active agent into the cytosol of human cells is combined with the active agent.

7. Composition according to claim 6, whereby the first carrier medium is a carrier protein, a cell penetrating peptide, a polar aprotic solvent such as Dimethyl sulfoxide (DMSO), a lipid, an alcohol, a permease, a liposome, a sulfur containing compound or a mixture thereof.

8. Composition according to claim 6, whereby the second pharmaceutically acceptable carrier medium to enable and facilitate the application and absorption of the active agent is added to the composition such as a liquid carrier, if the active agent shall be injected, a gelatin for sublingual application, a suppository for anal, a cream, spray or oil for mucosal or transdermal application and a syrup or substance for producing pills or capsule for oral application.

9. Composition according to claim 6, 7 or 8, whereby the weight ratio of the active agent to the carrier substance is preferably in the range between 1:10 and 1:250, most preferably at 1:33.

10. Group of at least a first composition according to one of the claims 1 - 8 and a second composition according to one of the claims 1 - 8 that comprise different carrier media and that are applicable in different parts of the body for forwarding the active agent via different pathways to a predetermined application zone, whereby the first composition that is provided with a first concentration of the active agent and the second composition that is provided with a second concentration of the active agent are adapted to one another in such a way that the desired amount of active agent is delivered to the field of application.

11. Group of at least a first and a second composition according claim 10, wherein one of said compositions is applied directly or indirectly on the inner side or the blood vessel side of a human organ and the other one of said compositions is applied directly or indirectly on the other or exterior side of the same human organ.

12. Group of at least a first and a second composition according claim 11, wherein the first composition is provided for transdermal application and the second composition is provided for inhalatory application.

13. Group of at least a first and a second composition according claim 11, wherein the first composition is provided for external or transdermal application and the second composition is provided for oral application.

14. Group of at least a first and a second composition according to one of the claims 10 to 13, wherein the first and the second composition comprise active agents that differ from one another in composition and/or concentration.
